Europäisches Patentamt

European Patent Office

Office européen des brevets

(19)

(11) Veröffentlichungsnummer: **0 178 553**
**B1**

(12) **EUROPÄISCHE PATENTSCHRIFT**

(45) Veröffentlichungstag der Patentschrift: **12.09.90**

(21) Anmeldenummer: **85112563.3**

(22) Anmeldetag: **04.10.85**

(51) Int. Cl.⁵: **C 12 P 13/22,** C 12 P 41/00,
C 07 B 55/00

(54) **Verfahren zur Herstellung aromatisch substituierter L-Aminosäuren.**

(30) Priorität: **18.10.84 DE 3438189**

(43) Veröffentlichungstag der Anmeldung:
**23.04.86 Patentblatt 86/17**

(45) Bekanntmachung des Hinweises auf die
Patenterteilung:
**12.09.90 Patentblatt 90/37**

(84) Benannte Vertragsstaaten:
**AT BE CH DE FR GB IT LI LU NL SE**

(56) Entgegenhaltungen:
**US-A-3 813 317**
**US-A-3 878 043**

**BIOTECHNOLOGY AND BIOENGINEERING, vol.
XIX, 1977, Seiten 1667-1677, John Wiley &
Sons; W.HAWACHS et al.: "Application of
immobilized chymotrypsin in a multistage
fluidized-bed reactor"**

(73) Patentinhaber: **HOECHST
AKTIENGESELLSCHAFT**
**Postfach 80 03 20**
**D-6230 Frankfurt am Main 80 (DE)**

(72) Erfinder: **Keller, Reinhold, Dr.**
**Wiesenweg 5**
**D-6232 Bad Soden am Taunus (DE)**
Erfinder: **Schlingmann, Merten, Dr.**
**Schneidhainer Strasse 32a**
**D-6240 Königstein/Taunus (DE)**

Courier Press, Leamington Spa, England.

EP 0 178 553 B1

# Beschreibung

Aus der deutschen Offenlegungsschrift 2 215 853 ist es bekannt, racemische mono- und di-ringsubstituierte Phenylalanine in die entsprechenden L-Aminosäuren dadurch zu überführen, daß man das Racemat zunächst mit einem Alkanol mit bis zu 4 Kohlenstoffatomen verestert, den racemischen Ester der Einwirkung eines Chymotrypsins bei einem sauren pH unterwirft, die L-Aminosäure durch Fällung isoliert und gegebenenfalls den Ester der D-Aminosäure aus dem Filtrat extrahiert und verseift. Als Enzym wird das α-Chymotrypsin bevorzugt, der bevorzugte pH-Bereich bei der enzymatischen Spaltung liegt bei 5 bis 6. Die nach Abtrennung der L-Aminosäure verbleibende Flüssigkeit wird vor der Extraktion des Esters der D-Aminosäure alkalisch gestellt.

Es wurde nun gefunden, daß aromatisch substituierte L-Aminosäuren besonders vorteilhaft dadurch zugänglich werden, daß das bei der enzymatischen Spaltung erhaltene Gemisch zunächst alkalisch gestellt wird, hierauf der Ester der D-Aminosäure extrahiert, gegebenenfalls nach Trocknung des Extrakts der Ester racemisiert und das Racemat in den Prozeß zurückgeführt wird. Aus dem alkalischen Rückstand nach Extraktion des Esters der D-Aminosäure wird dann die L-Aminosäure nach Ansäuern isoliert.

Die Erfindung betrifft somit ein Verfahren zur Herstellung von aromatisch substituierten L-Aminosäuren durch Spaltung der racemischen niederen Alkylester (ausgenommen tert.-Butylester) mit α-Chymotrypsin im schwach sauren wäßrigen Medium, das dadurch gekennzeichnet ist, daß das Reaktionsgemisch schwach alkalisch gestellt wird, der Ester der D-Aminosäure mit einem organischen Lösemittel extrahiert, der Ester racemisiert und wieder in den Prozeß zurückgeführt wird, und die L-Aminosäure aus dem wäßrigen alkalischen Extrakt nach Ansäuern isoliert wird. Bevorzugte Ausgestaltungen dieser Erfindung werden im folgenden näher erläutert bzw. in den Patentansprüchen definiert.

Als aromatisch substituierte Aminosäuren kommen vor allem Tryptophan, insbesondere Phenylalanin, Tyrosin und 3,4-Dihydroxy-Phenylalanin in Betracht.

Die Veresterung der racemischen Aminosäuren kann nach den Angaben der deutschen Offenlegungsschrift 2 215 853 erfolgen. Bevorzugt sind niedere Alkanole mit bis zu 4 Kohlenstoffatomen (ausgenommen tert.-Butanol), insbesondere Methanol, Ethanol, n-Propanol, Isopropanol und n-Butanol.

Das α-Chymotrypsin wird vorteilhaft in fixierter Form eingesetzt, wobei anorganische Träger bevorzugt sind. Dieses fixierte Enzym wird dann im Fest- oder Fließbett mit der wäßrigen Substratlösung in Kontakt gebracht. Die Umsetzung erfolgt zweckmäßig bei 20 bis 45°C, vorteilhaft bei 30°C—40°C, und einem pH-Wert im Bereich von 4,5 bis 6,5, vorteilhaft bei einem konstanten pH-Wert im Bereich von 5,0 bis 6,0, je nach eingesetztem Ester. Die Substratkonzentration ist an sich unkritisch, zweckmäßig sind 5—15 Gew.-%, insbesondere 10 bis 15 Gew.-%.

Die nach der enzymatischen Spaltung erhaltene wäßrige Lösung der L-Aminosäure und des Esters der D-Aminosäure wird schwach alkalisch gestellt, vorteilhaft auf einen pH-Wert im Bereich von 7,5 bis 8,5, vorteilhaft 8,0, und — zweckmäßig bei 20 bis 40°C, insbesondere Raumtemperatur — mit einem geeigneten organischen Lösemittel extrahiert.

Als organisches Lösemittel für die Extraktion wählt man zweckmäßig ein solches, das wenig wassermischbar ist, da dann die Racemisierung besonders vorteilhaft ohne Isolierung des Esters erfolgen kann (die Anwesenheit von Wasser bei der Racemisierung kann Nebenreaktionen wie Hydrolyse bedingen). Vorteilhaft ist deshalb ein Lösemittel, des mit Wasser ein Azeotrop bildet und hierdurch eine leichte Trocknung des Extraktes erlaubt. Es ist aber auch möglich, wenn auch im allgemeinen nicht vorteilhaft, das zur Extraktion benutzte Lösemittel völlig abzutrennen und den Ester in Substanz zu racemisieren.

Geeignete Lösemittel für die Extraktion sind aliphatische Kohlenwasserstoffe wie Hexan, insbesondere aromatische Kohlenwasserstoffe wie Toluol oder Xylol, Ether, insbesondere niedere Dialkylether wie Diethylether, Diisopropylether oder Di-n-butylether, Ketone, insbesondere niedere Dialkylketone wie Methyl-Ethyl-Keton oder Methyl-Isobutyl-Keton, halogenierte Kohlenwasserstoffe, insbesondere chlorierteniedere Aliphaten wie Methylenchlorid, Chloroform oder Tetrachlormethan sowie Ester, insbesondere niedere Alkylester niederer aliphatischer Carbonsäuren wie Ethylacetat oder Butylacetat.

Die Racemisierung kann in an sich bekannter Weise durch Ketoverbindungen und/oder Säuren katalysiert werden. Falls also das Lösemittel kein Keton ist, kann ein Zusatz von Aldehyden oder Ketonen die Racemisierung wesentlich beschleunigen. Die Racemisierung optisch aktiver Aminosäureester in Ketonen, ggfs. unter Zusatz einer Säure, ist aus der veröffentlichten japanischen Patentanmeldung 109 912/1979 bekannt. Bei dieser bekannten Racemierungsreaktion kann auch ein Lösemittel wie Toluol, Tetrachlormethan oder Methanol zugesetzt werden. Die Racemisierung freier optisch aktiver Aminosäuren mit Aldehyden und Säuren ist bekannt aus der veröffentlichten europäischen Patentanmeldung 57 092 sowie aus S. Yamada et al., J. Org. Chem. 48 (1983) 843—846. Zweckmäßig werden diese Ketoverbindungen in katalytischen Mengen, bevorzugt 0,001 bis 0,1 Mol pro Mol Ester, eingesetzt. Bevorzugt sind Acetaldehyd und Salicylaldehyd.

Das bei der Racemisierung entstandene D-, L-Gemisch wird dann mit leicht angesäuertem Wasser aus dem Racemisierungsansatz extrahiert, wobei der Ester in die Salzform überführt wird. Diese wäßrige Lösung wird durch Zugabe von neuem Substrat auf die gewünschte Konzentration, vorteilhaft 5 bis 15, insbesondere 10 bis 15 Gew.-%, eingestellt und in den Prozeß zurückgeführt.

Aus dem schwach alkalischen wäßrigen Rückstand, der die L-Aminosäure enthält, wird diese durch Ansäuern isoliert. Je nach Löslichkeit der Säure kann sie unmittelbar oder nach Konzentration kristallisieren. Die Säure kann auch in bekannter Weise durch Extraktion mit geeigneten Lösemitteln isoliert werden. Die Reinigung erfolgt in bekannter Weise.

Das erfindungsgemäße Verfahren erlaubt die Gewinnung von aromatisch substituierten L-Aminosäuren in Ausbeuten von über 90% und in einer optischen Reinheit von über 98%. Durch die Kombination von enzymatischer Spaltung, Extraktion des D-Esters, dessen Racemisierung und Rückführung ist eine besonders zweckmäßige Verbindung dieser Arbeitsschritte gegeben, die auch eine kontinuierliche Ausgestaltung des Verfahrens erlaubt.

In den folgenden Beispielen wird die Erfindung näher erläutert. Prozentangaben beziehen sich auf das Gewicht, sofern keine anderen Angaben gemacht sind.

### Beispiel 1
### Immobilisierung von α-Chymotrypsin

Die Fixierung des Enzyms kann auf Aluminiumsilikat nach Halwachs et al., Biotechnology and Bioengineering XIX (1977) 1667—1677 erfolgen. Besonders bevorzugt ist jedoch die folgende Arbeitsweise:

138 g trockenes Kieselgel (Korngröße 0,1 bis 0,3 mm) werden in 1 l 3,5%iger acetonischer 3-Aminopropyl-triethoxysilan-Lösung aufgenommen und 2 Stunden bei Raumtemperatur gelinde gerührt. Anschließend wird das Aceton in einem Rotationsverdampfer abgezogen und das Kieselgel im Vakuumtrockenschrank 8 Stunden bei 100°C getrocknet. Das Produkt enthält 0,89 mMol Aminogruppen pro Gramm Kieselgel. Dieses aminierte Kieselgel wird in 400 ml 25%iger wäßriger Glutardialdehyd-Lösung aufgenommen und 2 Stunden bei Raumtemperatur unter Vakuum (etwa 130 mbar) gehalten. Anschließend wird der Träger abfiltriert und gründlich mit entsalztem Wasser nachgewaschen.

138 g des so aktivierten Kieselgels werden in eine Lösung von 13,8 g α-Chymotrypsin (Firma Novo, 800 S Oral Grade) in 300 ml 0,5 m Phosphat-Puffer (pH 7,5) eingetragen und 4 Stunden bei Raumtemperatur gerührt. Anschließend wird der Katalysator abfiltriert und jeweils mit 1 l entsalztem Wasser, gesättigter Kochsalzlösung und entsalztem Wasser gewaschen. Zur Aufbewahrung wird das immobilisierte α-Chymotrypsin in 500 ml 0,1 m Phosphat-Pufferlösung (pH 7,5, 0,1 mMol Natriumazid) aufgenommen.

Zur Aktivitätsbestimmung gibt man 1 g des immobilisierten α-Chymotrypsins zu 50 ml einer 10%igen wäßrigen Lösung von D,L-Phenylalaninmethylester-Hydrochlorid, eingestellt auf pH 6,0 und thermostatisiert auf 30°C, und rührt diese Lösung. Der pH-Wert wird durch Zugabe von 0,1 N Natronlauge über eine automatische Bürette konstant gehalten. Die Menge an verbrauchter Natronlauge ist die Meßgröße und direkt proportional der Konzentration an L-Phenylalanin.

Aktivität: 338 U/g Träger (trocken) = 3,35 kg L-Phenylalanin/kg Träger und h (U = internationale Einheit für Enzymaktivität, 1 U = 1 μMol/Minute Umsatz).

### Beispiel 2
### Enzymatische Racematspaltung

In ein thermostatisierbares Reaktionsgefäß, versehen mit Innenrührer, pH-Elektrode und automatischer Bürette, werden 100 g D,L-Phenylalaninmethylester-Hydrochlorid (Restgehalt an D,L-Phenylalanin unter 0,3%, HPLC-Nachweisgrenze) gegeben und in 1 l entsalztem Wasser gelöst. Der pH-Wert der Lösung wird mit 5 N Natronlauge (9,5 ml) auf 6,0 eingestellt. Die Lösung wird auf 30°C thermostatisiert und mit einer Durchflußgeschwindigkeit von 5,0 l pro Stunde im Kreislauf über eine Säule gepumpt, die mit 150 ml des nach Beispiel 1 fixierten Enzyms gefüllt ist (5,0 cm Durchmesser, 8 cm Betthöhe).

Der pH-Wert der Lösung wird während der Reaktion durch Zugabe von 5 N Natronlauge (37,8 ml) auf 6,0 gehalten. Nach 25 Minuten Reaktionszeit ist laut HPLC-Analyse ein Umsatz von 49,2%, bezogen auf D,L-Phenylalaninmethylester, erzielt. Hierauf wird die Reaktion abgebrochen und das Enzymbett mit 300 ml entsalztem Wasser gewaschen.

### Beispiel 3
### Extraktion von D-Phenylalaninmethylester und anschließende Racemisierung

Die nach Beispiel 2 erhaltene wäßrige Lösung von D-Phenylalaninmethylester und L-Phenylalanin wird unter Rühren durch Zugabe von 5 N Natronlauge (36,5 ml) auf pH 8,0 gestellt und bei Raumtemperatur dreimal mit je 300 ml Methylisobutylketon ausgeschüttelt. Nach der dritten Extraktion ist in der wäßrigen Phase laut HPLC-Analyse kein Ester mehr enthalten (Nachweisgrenze: unter 0,2%). Die vereinigten Extrakte werden mit 14,0 g Eisessig versetzt (0,5 Mol-Äquivalent, bezogen auf D-Ester) und das im Extrakt enthaltene Wasser über eine Vigreux-Kolonne azeotrop abdestilliert. Der trockene Extrakt wird weitere zwei Stunden unter Rückfluß erhitzt, wobei der Racemisierungsgrad durch Bestimmung des optischen Drehwertes der Lösung mit Hilfe eines Polarimeters ermittelt wird. Die Lösung wird nach Abkühlen auf Raumtemperatur dreimal mit je 300 ml 0,05 N Salzsäure ausgeschüttelt. In den vereinigten wäßrigen Phasen ist laut HPLC-Analyse 98,5% des eingesetzten Esters enthalten. Diese wäßrige Lösung wird durch Zugabe von D,L-Phenylalaninmethylester-Hydrochlorid auf 10% eingestellt und für eine weitere enzymatische Spaltung eingesetzt.

Werden als Extraktionsmittel n-Hexan, Di-n-butylether, Toluol oder Xylol eingesetzt, so werden zur Beschleunigung der Reaktion katalytische Mengen Acetaldehyd, Salicylaldehyd oder Benzaldehyd zugegeben.

Anstelle des Eisessigs können auch Salzsäure, Benzoesäure, Zitronensäure, p-Toluolsulfonsäure oder Ameisensäure eingesetzt werden. Ohne Säuren verläuft die Racemisierung sehr viel langsamer und erfordert mehr als 10 Stunden. Säurekonzentrationen über 0,5 Mol-Äquivalent bewirken keine weitere Geschwindigkeitserhöhung.

### Beispiel 4
Aufarbeitung und Isolierung von L-Phenylalanin

Die nach der Extraktion mit Methylisobutylketon nach Beispiel 3 resultierende Lösung, die ca. 3,5% L-Phenylalanin enthält, wird mit konzentrierter Salzsäure auf pH 5,5 (isoelektrischer Punkt von Phenylalanin) eingestellt und in einem Rotationsverdampfer auf einen Gehalt von etwa 10% L-Phenylalanin eingeengt. Das ausgefallene L-Phenylalanin wird durch Erhitzen auf 90°C in Lösung gebracht und die Lösung heiß filtriert. Die klare Lösung läßt man bei Raumtemperatur stehen, wobei das L-Phenylalanin in großen Kristallen ausfällt. Die Mutterlauge wird über Nacht bei +5°C aufbewahrt, wobei weiße Kristalle ausfallen, die abfiltriert und mit wenig kaltem Wasser nachgewaschen werden. Die Kristalle werden drei Stunden bei 105°C im Vakuumtrockenschrank getrocknet. Man erhält 35,3 g L-Phenylalanin oder 92%, bezogen auf 50 g eingesetztes L-Phenylalaninmethylester-Hydrochlorid. Die optische Reinheit liegt bei 98,5 bis 99,5%.

### Beispiel 5
Enzymatische Racematspaltung:
D,L-Phenylalaninethylester

Analog Beispiel 2 werden 150 g D,L-Phenylalaninethylester-Hydrogensulfat in 1 l entsalztem Wasser gelöst. Der pH-Wert der Lösung wird mit 5 N-Natronlauge auf 5,5 eingestellt. Die Lösung wird auf 40°C thermostatisiert und mit einer Durchflußgeschwindigkeit von 60 l/Stunde im Kreislauf über die Säule gepumpt, wobei während der gesamten Reaktion durch Zugabe von 5 N-Natronlauge der pH-Wert auf 5,5 gehalten wird.

Die Weiterverarbeitung verläuft entsprechend Beispiel 3 und 4.

### Beispiel 6
Enzymatische Racematspaltung:
D,L-Phenylalanin-n-propylester

Analog Beispiel 5 wird D,L-Phenylalanin-n-propylester-Hydrogensulfat umgesetzt, wobei jedoch der pH-Wert auf 5,3 eingestellt und gehalten wird. Die weitere Aufarbeitung erfolgt analog Beispiel 3 und 4.

Mit gleichem Erfolg kann auch der Isopropyl- oder n-Butylester des D,L-Phenylalanins eingesetzt werden.

### Beispiel 7
L-Tryptophan

Analog Beispiel 2 werden 50 g D,L-Tryptophanmethylester-Hydrochlorid in 1 l entsalztem Wasser gelöst und wie beschrieben enzymatisch gespalten. Aus der so erhaltenen wäßrigen Lösung wird nach Beispiel 3 der D-Tryptophanmethylester extrahiert und die zurückbleibende wäßrige Phase analog Beispiel 4 mit konzentrierter Salzsäure auf pH 5,9 (isoelektrischer Punkt von Tryptophan) eingestellt und in einem Rotationsverdampfer auf einen Gehalt von etwa 5% L-Tryptophan eingeengt. Die ausgefallenen Kristalle werden abfiltriert, mit kaltem Wasser nachgewaschen und bei 105°C 3 Stunden getrocknet. Man erhält 19,4 g oder 97%, bezogen auf 25 g eingesetztes L-Tryptophan Methylester-Hydrochlorid. Die optische Reinheit liegt bei 98,7%.

## Patentansprüche

1. Verfahren zur Herstellung von aromatisch substituierten L-Aminosäuren durch enzymatische Spaltung des Racemats der niederen Alkylester (ausgenommen tert.-Butylester) mit α-Chymotrypsin im schwach sauren pH-Bereich und Extraktion des Esters der D-Form, dadurch gekennzeichnet, daß das bei der enzymatischen Spaltung erhaltene Reaktionsgemisch schwach alkalisch gestellt wird, aus dieser alkalischen Lösung der Ester der D-Aminosäure mit einem organischen Lösemittel extrahiert und racemisiert wird, worauf das Racemat in den Prozeß zurückgeführt wird, und die extrahierte alkalische Lösung schwach sauer gestellt wird, worauf aus dieser die L-Aminosäure isoliert wird.

2. Verfahren nach Anspruch 1, dadurch gekennzeichnet, daß das α-Chymotrypsin in fixierter Form eingesetzt wird.

3. Verfahren nach Anspruch 2, dadurch gekennzeichnet, daß das α-Chymotrypsin an einen anorganischen Träger fixiert ist.

4. Verfahren nach einem oder mehreren der vorhergehenden Ansprüche, dadurch gekennzeichnet, daß zur Extraktion des D-Esters ein wenig wassermischbares organisches Lösemittel eingesetzt wird.

5. Verfahren nach Anspruch 4, dadurch gekennzeichnet, daß ein Lösemittel eingesetzt wird, das mit Wasser ein Azeotrop bildet.

6. Verfahren nach einem oder mehreren der vorhergehenden Ansprüche, dadurch gekennzeichnet, daß der gegebenenfalls getrocknete Extrakt des D-Esters der Racemisierung unterworfen wird.

7. Verfahren nach einem oder mehreren der vorhergehenden Ansprüche, dadurch gekennzeichnet, daß der racemisierte Ester sauer extrahiert und nach Aufstärken in den Prozeß zurückgeführt wird.

8. Verfahren nach einem oder mehreren der vorhergehenden Ansprüche, dadurch gekennzeichnet, daß der Ester ein Alkylester mit 1 bis 4 Kohlenstoffatomen, ausgenommen tert.-Butyl, ist.

9. Verfahren nach einem oder mehreren der vorhergehenden Ansprüche, dadurch gekennzeichnet, daß der Ester ein Methyl-, Ethyl-, n-Propyl-, Isopropyl- oder n-Butylester des Phenylalanins oder Tryptophans ist.

**Revendications**

1. Procédé pour la préparation de L-aminoacides aromatiques substitués, par dédoublement enzymatique du racémique des esters alkyliques inférieurs (à l'exception de l'ester tert-butylique) avec de l'α-chymotrypsine à pH faiblement acide, et extraction de l'ester de la forme D, caractérisé en ce que l'on alcalinise légèrement le mélange réactionnel obtenu après le dédoublement enzymatique, on extrait de cette solution alcaline, à l'aide d'un solvant organique, l'ester du D-aminoacide et on le racémise, puis on recycle le racémique dans le processus et on acidifie légèrement la solution alcaline extraite, à la suite de quoi on isole le L-aminoacide hors de celle-ci.

2. Procédé selon la revendication 1, caractérisé en ce que l'on utilise l'α-chymotrypsine sous forme fixée.

3. Procédé selon la revendication 2, caractérisé en ce que l'α-chymotrypsine est fixée sur un support minéral.

4. Procédé selon une ou plusieurs des revendications précédentes, caractérisé en ce que, pour l'extraction du D-ester, on utilise un solvant organique peu miscible à l'eau.

5. Procédé selon la revendication 4, caractérisé en ce que l'on utilise un solvant qui forme un azéotrope avec l'eau.

6. Procédé selon une ou plusieurs des revendications précédentes, caractérisé en ce que l'on soumet à la racémisation l'extrait du D-ester éventuellement séché.

7. Procédé selon une ou plusieurs des revendications précédentes, caractérisé en ce que on extrait à pH acide l'ester après racémisation et on le recycle dans le processus après enrichissement.

8. Procédé selon une ou plusieurs des revendications précédentes, caractérisé en ce que l'ester est un ester alkylique ayant de 1 à 4 atomes de carbone, à l'exception de l'ester tert-butylique.

9. Procédé selon une ou plusieurs des revendications précédentes, caractérisé en ce que l'ester est l'ester méthylique, éthylique, n-propylique, isopropylique ou n-butylique de la phénylalanine ou du tryptophane.

**Claims**

1. A process for the preparation of aromatically substituted L-amino acids by enzymatic cleavage of the racemate of the lower alkyl esters (except tert.-butylester) using α-chymotrypsin in the weakly acid pH range and extraction of the ester of the D form, which comprises the reaction mixture obtained in the enzymatic cleavage being made weakly alkaline, the ester of the D-amino acid being extracted from this alkaline solution using an organic solvent, and being racemized, whereupon the racemate is returned to the process, and the extracted alkaline solution being made weakly acid, whereupon the L-amino acid is isolated from it.

2. The process as claimed in claim 1, wherein the α-chymotrypsin is used in the immobilized form.

3. The process as claimed in claim 2, wherein the α-chymotrypsin is immobilized on an inorganic carrier.

4. The process as claimed in one or more of the preceding claims, wherein an organic solvent of low miscibility with water is used for the extraction of the D-ester.

5. The process as claimed in claim 4, wherein the solvent used forms an azeotrope with water.

6. The process as claimed in one or more of the preceding claims, wherein the extract, where appropriate dried, of the D-ester is subjected to the racemization.

7. The process as claimed in one or more of the preceding claims, wherein the racemized ester is extracted under acid conditions and, after concentration, is returned to the process.

8. The process as claimed in one or more of the preceding claims, wherein the ester is an alkyl ester having 1 to 4 carbon atoms, excepting tert.-butyl.

9. The process as claimed in one or more of the preceding claims, wherein the ester is a methyl, ethyl, n-propyl, isopropyl or n-butyl ester of phenylalanine or tryptophan.